(19) Europäisches Patentamt / European Patent Office / Office européen des brevets

(11) **EP 3 561 446 A1**

(12) **EUROPÄISCHE PATENTANMELDUNG**

(43) Veröffentlichungstag:
**30.10.2019 Patentblatt 2019/44**

(51) Int Cl.:
*G01B 11/245* [(2006.01)]     *G01M 11/02* [(2006.01)]

(21) Anmeldenummer: **18168823.5**

(22) Anmeldetag: **23.04.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Carl Zeiss Vision International GmbH 73430 Aalen (DE)**

(72) Erfinder: **Glasenapp, Carsten 73447 Oberkochen (DE)**

(74) Vertreter: **Witte, Weller & Partner Patentanwälte mbB Postfach 10 54 62 70047 Stuttgart (DE)**

(54) **VERFAHREN UND VORRICHTUNG ZUM VERMESSEN EINER OPTISCHEN LINSE FÜR INDIVIDUELLE TRAGESITUATIONEN EINES NUTZERS**

(57)     Die vorliegende Erfindung betrifft das Gebiet der Augenoptik und insbesondere eine Vorrichtung (10) zum Vermessen der optischen Wirkung einer in einem Messvolumen (200) angeordneten optischen Linse (100), insbesondere eines Brillenglases, mit einer Anzeigeeinrichtung (20) eingerichtet zum Anzeigen einer Teststruktur (21); einer Bilderfassungseinrichtung (30), eingerichtet zum Erfassen von Bilddaten der Teststruktur aus mehreren Blickpunkten (31, 31', 31") durch Abbildungsstrahlengänge (32), welche die Linse (100) durchsetzen; und einer Recheneinheit (40), wobei die Recheneinheit eingerichtet ist zum: Bestimmen einer dreidimensionalen Form der Linse (100) basierend auf den Bilddaten; und Berechnen einer optischen Wirkung der Linse (100) basierend auf deren dreidimensionaler Form. Die vorliegende Erfindung betrifft ferner ein entsprechendes Verfahren und Computerprogramm.

Fig. 1

EP 3 561 446 A1

**Beschreibung**

Die vorliegende Erfindung betrifft das Gebiet der Augenoptik und insbesondere eine Vorrichtung zum Vermessen der optischen Wirkung einer in einem Messvolumen angeordneten optischen Linse, insbesondere eines Brillenglases. Die vorliegende Erfindung betrifft ferner eine Vorrichtung zum Vermessen einer räumlichen Brechzahlverteilung einer in einem Messvolumen angeordneten optischen Linse, insbesondere eines Brillenglases. Die vorliegende Erfindung betrifft ferner ein Verfahren zum Kalibrieren einer entsprechenden Vorrichtung, ein computerimplementiertes Verfahren zum Vermessen der optischen Wirkung einer in einem Messvolumen angeordneten optischen Linse sowie ein entsprechendes Computerprogramm.

Der üblicherweise interessante Messwert bei Brillengläsern ist der Scheitelbrechwert (SBW). Der Scheitelbrechwert ist eine Wirkgröße der Linse unter einer bestimmten Beobachtungssituation. Folglich ist der SBW unterschiedlich je nachdem wie groß der Abstand zum Betrachter ist oder wie die Linse geneigt ist. Ein Messgerät, welches einen SBW durch direkte Interpretation der durch eine Linse durchdringenden Lichtstrahlen ermittelt, wird immer einen SBW dieser Messgerätekonfiguration bestimmen. Für eine eindeutige Qualifizierung eines Bauteiles sind solche Wirkgrößen nur bedingt nützlich. Als Abhilfe wurde deshalb der ISO SBW definiert. Der ISO SBW ist der SBW, welcher senkrecht zu der Flächennormale bei parallelem Lichteinfall gemessen wird. Hierzu wurden in der Vergangenheit spezielle Messgeräte entwickelt, welche an einzelnen Positionen einer Linse den ISO SBW bestimmen.

Der Vorteil des ISO SBW ist, dass dieser eine eindeutige Bauteilgröße ist und nicht wie der SBW eine Wirkgröße. Nachteilig ist, dass der ISO SBW abweichen kann von der Wirkung einer Brille in der Trägersituation (auch als Gebrauchs-Scheitelbrechwert oder Gebrauchswert bezeichnet).

Ein Scheitelbrechwertmesser mit einer Brillenanlage für die Nachprüfung fertig verglaster Brillen ist aus der DE 1 238 690 B1 bekannt. Damit kann der Scheitelbrechwert eines Brillenglases, welches bereits in einer Fassung eingefasst ist, bestimmt werden.

Aus der WO 2016/207412 A1 sind eine Vorrichtung und ein Verfahren zum Vermessen von individuellen Daten einer in einer Messposition angeordneten Brille mit einem linken und/oder einem rechten Brillenglas bekannt. Die Vorrichtung weist ein Display für das Anzeigen einer Teststruktur auf. Die Vorrichtung weist eine Bilderfassungseinrichtung für das Erfassen der Teststruktur mit einem Abbildungsstrahlengang auf, der das linke Brillenglas und/oder das rechte Brillenglas der Brille durchsetzt. Die Vorrichtung hat eine Rechnereinheit mit einem Computerprogramm, das aus der mit der Bilderfassungseinrichtung erfassten Abbildung der Teststruktur und einer bekannten räumlichen Lage des Displays relativ zu der Bilderfassungseinrichtung sowie einer bekannten räumlichen Lage der Brille relativ zu der Bilderfassungseinrichtung eine Brechkraftverteilung für wenigstens einen Abschnitt des linken Brillenglases und/oder des rechten Brillenglases bestimmt.

Bei aus dem Stand der Technik bekannten Geräten handelt es sich um Wirkungsmessgeräte, bei welchen zunächst die Wirkung eines optischen Elements in einer Messposition bestimmt wird.

Vor diesem Hintergrund ist es eine Aufgabe der vorliegenden Erfindung, eine Messvorrichtung bereitzustellen, welche eine flexiblere Bestimmung der optischen Wirkung einer optischen Linse ermöglicht.

Gemäß einem ersten Aspekt der vorliegenden Offenbarung wird daher vorgeschlagen, eine Vorrichtung zum Vermessen der optischen Wirkung einer in einem Messvolumen angeordneten optischen Linse, insbesondere eines Brillenglases, bereitzustellen mit einer Anzeigeeinrichtung eingerichtet zum Anzeigen einer Teststruktur; einer Bilderfassungseinrichtung, eingerichtet zum Erfassen von Bilddaten der Teststruktur aus mehreren Blickpunkten durch Abbildungsstrahlengänge, welche die Linse durchsetzen; und einer Recheneinheit, wobei die Recheneinheit eingerichtet ist zum Bestimmen einer dreidimensionalen Form der Linse basierend auf den Bilddaten; und Berechnen einer optischen Wirkung der Linse basierend auf deren dreidimensionaler Form.

Gegenüber konventionellen Scheitelbrechwertmessgeräten können wesentliche Vorteile der Erfindung insbesondere in einer verbesserten Eindeutigkeit und/oder Einsatzbreite bestehen. Ein Grund hierfür ist, dass die optische Wirkung einer Linse immer abhängig von der Durchstrahlrichtung ist. Ein Messgerät, welches ausschließlich die optische Wirkung misst, kann diese nur sicher für den Fall der Messanordnung bestimmen. Für eine Vielzahl an Anwendungsfällen können hiermit bereits sehr genaue Aussagen getroffen werden.

Allerdings können eine Messsituation und eine tatsächliche Tragesituation oder Trageposition eines Brillenglases auseinanderfallen bzw. in einem Maß voneinander abweichen, dass eine verlässliche Aussage nicht mehr möglich ist. Um die optische Wirkung in der Trageposition zu bestimmen, wäre also eine weitere Wirkungsmessung unter Tragebedingungen erforderlich.

Die erfindungsgemäße Lösung verfolgt einen anderen Ansatz: Es wird ein zweistufiges Vorgehen vorgeschlagen, bei welchen zunächst die dreidimensionale Form der Linse bestimmt wird und erst nachfolgend die optische Wirkung der Linse berechnet wird. Eine bekannte dreidimensionale Form bzw. Topographie der Linse ermöglicht es, dass die optische Wirkung nachfolgend für beliebige Durchblicks- oder Tragesituation berechnet werden kann. Vorteile können insbesondere genauere Ergebnisse und besser individualisierte Aussagen für eine große Bandbreite an nutzerspezifischen Wünschen umfassen.

Die Anzeigeeinheit zeigt eine Teststruktur an. Die Teststruktur wird von der Bilderfassungseinrichtung aus mehreren Blickpunkten erfasst. Da die Teststruktur bekannt ist, kann eine Zuordnung zwischen von der Bilderfassungseinrichtung erfassten Bilddaten der Teststruktur für jeden der mehreren Blickpunkte erfolgen. Wird nun eine optische Linse in einem Messvolumen zwischen der Anzeigeeinrichtung und der Bilderfassungseinrichtung platziert, so werden die Strahlengänge zwischen jeweiligen Pixeln der Bilddaten und den korrespondieren Bildelementen der Teststruktur beeinflusst.

Hierbei lässt sich jedoch nicht nur eine einzelne virtuelle Brechebene, wie in der WO 2016/207412 A1 angedeutet, bestimmen. Indem gemäß der vorgeschlagenen Lösung Bilddaten aus mehreren Blickpunkten erfasst werden, wobei die Abbildungsstrahlengänge die Linse durchsetzen, können insbesondere separate Aussagen über eine Form einer Vorderfläche, durch welche von der Teststruktur ausgehende Strahlengänge in die optische Linse eintreten, und Aussagen über eine Form einer Rückfläche, über welche von der Teststruktur ausgehende Strahlengänge aus der optischen Linse austreten, getroffen werden. Es kann hierfür also ein Gleichungssystem mit einer Vielzahl an Gleichungen aufgestellt werden, auf deren Basis eine Rekonstruktion der im Strahlengang liegenden Flächen erfolgen kann. Die dreidimensionale Form der Linse folgt wiederum aus der Form der Vorderfläche und der Form der Rückfläche.

Die Berechnung der optischen Wirkung basierend auf der dreidimensionalen Form kann nachfolgend mit bekannten Verfahren erfolgen.

Es versteht sich, dass nicht notwendigerweise die dreidimensionale Form der gesamten Linse bestimmt werden muss. Beispielsweise kann die Berechnung nur für einen Teilbereich erfolgen, beispielsweise nur die Vorder- und Rückfläche, ohne Seitenflächen oder nur einen Teilbereich im Sichtfeld eines Nutzers.

Bei der Bestimmung der dreidimensionalen Form der Linse durch die Recheneinheit können weitergehenden Informationen, wie beispielsweise eine bekannte räumliche Lage der Anzeigeeinrichtung relativ zu jeweiligen den Blickpunkten, aus welchen die Erfassung erfolgt, vorteilhaft berücksichtigt werden.

Gemäß einem zweiten Aspekt der vorliegenden Offenbarung wird eine Vorrichtung zum Vermessen einer räumlichen Brechzahlverteilung einer in einem Messvolumen angeordneten optischen Linse, insbesondere eines Brillenglases, vorgeschlagen mit einer Anzeigeeinrichtung eingerichtet zum Anzeigen einer Teststruktur; einer Bilderfassungseinrichtung eingerichtet zum Erfassen von Bilddaten der Teststruktur aus mehreren Blickpunkten durch Abbildungsstrahlengänge, welche die Linse durchsetzen; einer Schnittstelle eingerichtet zum Empfangen von Linsengeometriedaten, welche eine dreidimensionale Form der Linse beschreiben; und einer Recheneinheit, wobei die Recheneinheit eingerichtet ist zum Berechnen einer räumlichen Brechzahlverteilung der Linse basierend auf den Bilddaten und den Linsengeometriedaten.

Ein Vorteil dieser Lösung besteht darin, dass eine dreidimensionale Brechkraftverteilung innerhalb der Linse bestimmt werden kann. Beispielsweise zur Beschreibung eines Gleitsichtglases, bei welchem die optische Wirkung für Fern- und Nahbereich durch eine dreidimensionale Variation des Brechungsindices bereitgestellt wird. Optional können auch die Geometriedaten berechnet werden. In diesem Fall ist keine Schnittstelle zum Empfangen der Geometriedaten erforderlich.

Gemäß einem dritten Aspekt der vorliegenden Offenbarung wird ein Verfahren zum Kalibrieren einer Vorrichtung zum Vermessen von individuellen Daten einer in einem Messvolumen angeordneten optischen Linse bereitgestellt, wobei das Verfahren folgende Schritte aufweist: Bereitstellen bzw. Anzeigen einer Teststruktur auf der Anzeigeeinrichtung; Einstellen eines ersten Abstandes zwischen der Bilderfassungseinrichtung und der Anzeigeeinrichtung und Erfassen von Bilddaten der Teststruktur mit der Bilderfassungseinrichtung aus dem ersten Abstand; Einstellen eines zweiten Abstandes zwischen der Bilderfassungseinrichtung und der Anzeigeeinrichtung und Erfassen von Bilddaten der Teststruktur mit der Bilderfassungseinrichtung aus dem zweiten Abstand; Bestimmen einer Richtung von einfallenden Lichtstrahlen, welche von der Bilderfassungseinrichtung erfasst werden, und korrespondierenden Bildpunkten in den Bilddaten, basierend auf den im ersten Abstand erfassten Bilddaten und den im zweiten Abstand erfassten Bilddaten.

Ein Vorteil dieser Lösung besteht darin, dass auf einfache Art und Weise eine Bestimmung der Richtung von einfallenden Lichtstrahlen erfolgen kann. Hierbei kann die ohnehin für die Messung verwendete Anzeigeeinrichtung auch der Kalibrierung dienen. Die Relativposition der Anzeigeeinrichtung einschließlich deren Bildpunkte relativ zur Bilderfassungseinrichtung, können vorzugsweise bei der Kalibrierung berücksichtigt werden.

Durch die Höhenverstellung ändert sich der Winkel der einfallenden Lichtstrahlen relativ zur Bilderfassungseinrichtung, beispielsweise zu den Kameras der Bilderfassungseinrichtung. Durch die Relation zwischen einer bekannten Höhenänderung und einer damit einhergehenden Änderung einer Abbildung der Teststruktur in den Bilddaten kann eine Richtung des einfallenden Lichts bestimmt werden. Dies ermöglicht eine sogenannte "Backpropagation" der einfallenden Lichtstrahlen.

Gemäß einem vierten Aspekt der vorliegenden Offenbarung wird ein Verfahren, insbesondere computerimplementiertes Verfahren, zum Vermessen der optischen Wirkung einer in einem Messvolumen angeordneten optischen Linse, insbesondere eines Brillenglases, offenbart mit den Schritten: Bereitstellen einer Teststruktur zur Anzeige auf einer Anzeigeeinrichtung; Erfassen von Bilddaten der Teststruktur aus mehreren Blickpunkten durch Abbildungsstrahlengänge, welche die Linse durchsetzen; Bestimmen einer dreidimensionalen Form der Linse basierend auf Bilddaten; und Berechnen einer optischen Wirkung der Linse basierend auf deren dreidimensionaler Form.

Gemäß einem weiteren Aspekt der vorliegenden Offenbarung wird ein Computerprogrammprodukt vorgeschlagen, umfassend Befehle, die bei der Ausführung des Programms durch einen Computer diesen veranlassen, das vorstehend genannte Verfahren auszuführen. Es versteht sich, dass die Verfahrensschritte hierbei zur Ausführung durch einen Computer ausgebildet sind. Beispielsweise kann unter Erfassen von Bilddaten das Empfangen von Bilddaten verstanden werden. Der Begriff kann also als eine Übermittlung von, von einem physikalischen Bildsensor erzeugten, Messdaten verstanden werden. Die Bereitstellung der Teststruktur kann entsprechend durch Bereitstellen von Teststrukturdaten erfolgen. Die Daten können wiederum von einer Anzeigeeinrichtung angezeigt werden.

Das Bereitstellen der Teststruktur kann auch ein vorgelagerter Schritt sein, welcher nicht von dem Computerprogrammprodukt ausgeführt wird.

Falls nicht anderweitig spezifiziert, sollen die hierin verwendeten Begriffe im Sinne der Norm DIN EN ISO 13666:2012 des Deutschen Instituts für Normung e.V. verstanden werden.

Der Begriff Vorderfläche oder objektseitige Fläche bezeichnet gemäß Abschnitt 5.8 der Norm DIN EN ISO 13666:2012 die Fläche eines Brillenglases, die bestimmungsgemäß in der Brille vom Auge abgewandt liegt. Der Begriff Rückfläche oder augenseitige Fläche bezeichnet gemäß Abschnitt 5.19 der Norm DIN EN ISO 13666:2012 die Fläche eines Brillenglases, die bestimmungsgemäß in der Brille dem Auge zugewandt liegt. Alternativ hierzu kann der Begriff Vorderfläche im Rahmen der vorliegenden Offenbarung diejenige Fläche der Linse bezeichnen, welche der Anzeigeeinrichtung zugewandt ist. Entsprechend kann eine Rückfläche im Rahmend der vorliegenden Offenbarung diejenige Fläche bezeichnen, welche der Anzeigeeinrichtung abgewandt ist.

In einer Ausgestaltung kann vorgesehen sein, dass die Bilderfassungseinrichtung eine erste Kamera und eine zweite Kamera aufweist, wobei die erste Kamera zum Erfassen von ersten Bilddaten aus einem ersten Blickpunkt eingerichtet ist und die zweite Kamera zum Erfassen von zweiten Bilddaten aus einem zweiten Blickpunkt eingerichtet ist; und wobei die Recheneinheit eingerichtet ist zum Bestimmen der dreidimensionalen Form der Linse basierend auf den ersten und zweiten Bilddaten. Alternativ zur Verwendung von zwei Kameras kann die Erfassung der ersten und zweiten Bilddaten auch mittels einer Kamera aus verschiedenen Positionen erfolgen. Um die Kamera zwischen der ersten und der zweiten Position zu bewegen, kann eine Verfahreinrichtung bzw. eine Positioniereinrichtung vorgesehen sein.

In einer optionalen Weiterbildung können die erste Kamera und die zweite Kamera in einem Winkel zueinander angeordnet sein, so dass die Teststruktur von der ersten Kamera aus einem ersten Winkel und von der zweiten Kamera aus einem zweiten Winkel erfasst werden kann.

In einer Ausgestaltung kann vorgesehen sein, dass die Linse ein Brillenglas ist und die Berechnung der optischen Wirkung des Brillenglases für eine Trageposition eines Nutzers erfolgt. Ein Vorteil kann insbesondere darin bestehen, dass eine Berechnung der optischen Wirkung auch im Nachhinein für jede beliebige Trageposition des Nutzers erfolgen kann. Hierbei kann sich die Trageposition auch deutlich von einer Messposition, in welcher die Bilddaten erfasst werden unterscheiden. Insbesondere kann eine nutzerspezifische Anpassung und eine flexible; Berechnung von Gebrauchswerten erfolgen. Demgegenüber treffen konventionelle Scheitelbrechwertmessgeräte keine individualisierte Aussage für den Nutzer.

In einer Ausgestaltung kann vorgesehen sein, dass die Recheneinheit dazu eingerichtet ist, die dreidimensionale Form der Linse iterativ mittels eines Integrationsverfahrens zu bestimmen.

In einer weiteren Ausgestaltung kann vorgesehen sein, dass die Recheneinheit eingerichtet ist zum Bestimmen der dreidimensionalen Form der Linse basierend auf einer Rückverfolgung der in die Bilderfassungseinrichtung eintretenden Lichtstrahlen. Insbesondere können in die Bilderfassungseinrichtung eintretende Lichtstrahlen zu bekannten Ursprungsorten der auf der Anzeigeeinrichtung angezeigten Teststruktur zurückverfolgt werden. Insbesondere sind die Relativposition der Anzeigeeinrichtung und die Positionen bzw. Blickpunkten, aus welchen die Bilddaten erfasst werden, bekannt. Die Relativpositionen können optional auf Basis der vorstehend beschriebenen Kamerakalibrierung mittels Abstands- bzw. Höhenänderung ermittelt werden. Zur Bestimmung der dreidimensionalen Form der Linse können Verfahren wie beispielsweise Backpropagation oder inversen Raytracing zum Einsatz kommen. Vereinfacht gesprochen kann eine Flächenrekonstruktion der zu messenden Linse basierend auf einem Vergleich einer Soll-Position und einer Ist-Position eines oder mehrerer Elemente der Teststruktur im erfassten Bild erfolgen.

In einer Ausgestaltung kann das Bestimmen der dreidimensionalen Form der Linse eine Aufteilung einer Vorder- und/oder Rückfläche der Linse in Flächenelemente und eine Bestimmung einer Ausrichtung der Flächenelemente, insbesondere eine Bestimmung von Oberflächennormalen der Flächenelemente, umfassen. Diese Bestimmung kann insbesondere basierend auf einer Rückverfolgung der in die Bilderfassungseinrichtung eintretenden Lichtstrahlen vorgenommen werden. Mit anderen Worten kann für (jedes) einzelne Flächenelement(e) eine Ausrichtung der Oberfläche bestimmt werden. Beispielsweise kann eine Berechnung von Oberflächennormalen für einzelne Abschnitte bzw. Flächenelemente erfolgen.

In einer Weiterbildung kann die Recheneinheit dazu ausgebildet sein, basierend auf der Ausrichtung der Flächenelemente, eine dreidimensionale Form einer Vorderfläche und/oder einer Rückfläche der Linse zu bestimmen. Eine Oberfläche der Linse, z.B. die Vorder- oder Rückfläche kann aus einzelnen Flächenelementen zusammengesetzt werden. Vorzugsweise wird die Oberfläche derart zusammengesetzt, dass keine (signifikanten) Sprünge zwischen benachbarten Elementen entstehen.

In einer Ausgestaltung kann die Recheneinheit dazu eingerichtet sein, die dreidimensionale Form der Linse zu bestimmen unter Berücksichtigung der Randbedingung, dass eine Vorderfläche oder Rückfläche der Linse eine parametrisierbare Fläche, insbesondere eine Ebene, Sphäre, Torus oder ein Abschnitt davon ist. Ein Vorteil besteht in einer schnelleren Berechnung und/oder höherer Genauigkeit, da der Parameterraum durch Vorgabe von Randbedingungen reduziert wird

In einer Ausgestaltung kann vorgesehen sein, dass die Recheneinheit dazu eingerichtet ist, die dreidimensionale Form der Linse ferner unter Berücksichtigung eines bekannten oder mehrerer bekannten Auflagepunkte(s) der Linse zu bestimmen. Alternativ oder zusätzlich kann vorgesehen sein, dass die Recheneinheit dazu eingerichtet sein, die dreidimensionale Form der Linse unter Berücksichtigung einer Randbedingung zu bestimmen, wobei die Randbedingung bestimmt wird durch Auslesen von Information über die zu vermessende Linse, insbesondere durch Auslesen einer Markierung oder eines Codes auf der Linse. Vorteile können erneut in einer schnelleren und/oder genaueren Berechnung bestehen, da die Freiheitsgrade weiter reduziert werden. Es versteht sich, dass auch mehrere bekannten Auflagepunkte oder eine Linsenglas- oder Brillenhalterung berücksichtigt werden können. Als Code auf einer Linse kann beispielsweise eine Gravur, ein Marker bzgl. einer Krümmung, eines Materials, oder eines Brechungsindices ausgelesen und in der Berechnung berücksichtigt werden.

In einer Ausgestaltung kann vorgesehen sein, dass die Recheneinheit ferner eingerichtet ist zur Bestimmung eines Brechungsindex, insbesondere zur Bestimmung einer räumlichen Brechzahlverteilung, der zu vermessenden Linse. Eine Linse bzw. ein Brillenglas mit einem Brechungsindex kann als Spezialfall angesehen werden. Vorzugsweise ist der Brechungsindex zumindest in einem Teilabschnitt konstant. Ferner kann eine räumliche Brechzahlverteilung einer sogenannten GRIN-Linse (GRaded-INdex) bestimmt werden. Die Erfinder haben erkannt, dass die vorgeschlagene Lösung nicht nur zur Formerfassung, sondern auch zur Bestimmung des Brechungsindex, d.h. der innere Vermessung eines transparenten Körpers dienen kann. Beispielsweise kann eine innere Grenzfläche zwischen Bereichen mit unterschiedlichem Brechungsindex bestimmt werden. Mögliche Anwendungsfälle sind beispielsweise mehrteilige Linsen, Linsen mit Materialien mit unterschiedlichem Brechungsindex, Achromaten, optische Systeme oder Objektive.

In einer Ausgestaltung kann die Vorrichtung ferner eine Höhenverstelleinrichtung aufweisen, die dazu eingerichtet ist, einem Abstand zwischen der Bilderfassungseinrichtung und der Anzeigeeinrichtung zu variieren. Ferner kann die Recheneinheit dazu eingerichtet sein, basierend auf aus unterschiedlichen Abständen zwischen der Bilderfassungseinrichtung und der Anzeigeeinrichtung erfassten Bilddaten, eine Strahlrichtung der von der Bilderfassungseinrichtung erfassten Lichtstrahlen zu bestimmen. Somit kann auf einfache Art und Weise eine Zuordnung zwischen Pixel und Strahlrichtung erfolgen.

Die vorstehend für den ersten Aspekt der Erfindung ausführlich beschriebenen Vorteile gelten für die weiteren Aspekte der Erfindung entsprechend.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsformen der Erfindung sind in der Zeichnung dargestellt und werden in der nachfolgenden Beschreibung näher erläutert.

Fig. 1 zeigt eine schematische Darstellung einer Vorrichtung zum Vermessen der optischen Wirkung einer in einem Messvolumen angeordneten optischen Linse;

Fig. 2 zeigt eine beispielhafte Darstellung einer durch eine Linse aufgenommenen Teststruktur;

Fig. 3 zeigt eine beispielhafte Darstellung einer durch ein verkipptes Brillenglas aufgenommenen Teststruktur;

Fig. 4 zeigt eine Darstellung von Strahlengängen durch ein transparentes Objekt;

Fig. 5 zeigt eine Darstellung von Strahlengängen durch eine Linse;

Fig. 6 zeigt eine aus parametrierbaren Flächenelementen zusammengesetzte Linse;

Fig. 7 zeigt eine schematische Darstellung einer Vorrichtung zum Vermessen der optischen Wirkung einer in einem Messvolumen angeordneten optischen Linse;

Fig. 8 zeigt eine weitere Ausführungsform einer Vorrichtung zum Vermessen der optischen Wirkung einer in einem Messvolumen angeordneten optischen Linse;

Fig. 9 zeigt ein Flussdiagramm einer Ausgestaltung eines Verfahrens zum Vermessen der optischen Wirkung einer in einem Messvolumen angeordneten optischen Linse;

Fig. 10    zeigt ein detailliertes Flussdiagram einer Ausgestaltung solchen Verfahrens;

Fig. 11    zeigt ein Flussdiagramm einer Ausführungsform eines Verfahrens zum Kalibrieren;

Fig. 12    zeigt eine schematische Darstellung eines Auges;

Fig. 13    zeigt eine Abbildung einer Draufsicht auf das Auge mit einer Abbildung der Iris;

Fig. 14    zeigt eine schematische Darstellung einer Vorrichtung zur CorneaVermessung;

Fig. 15    zeigt die Zuordnung bzw. Korrelation von Bildmerkmalen in aus unterschiedlichen Blickpunkten aufgenommenen Bildern der Iris;

Fig. 16    zeigt weitere Korrelationen von Bildmerkmalen; und

Fig. 17    zeigt ein Flussdiagramm einer Ausführungsform eines Verfahrens zur Cornea-Vermessung.

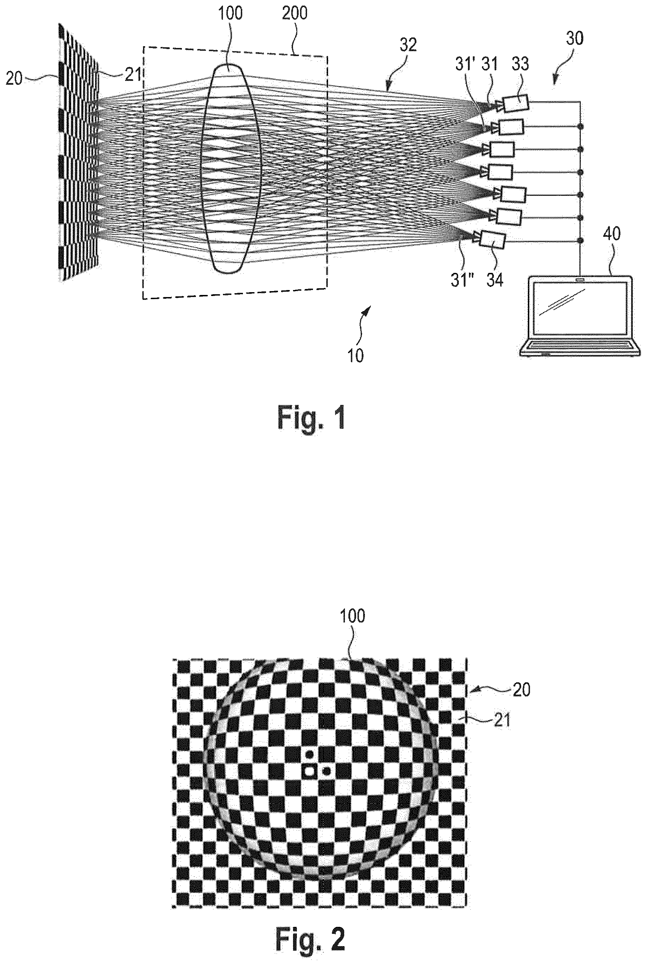

Die in Fig. 1 gezeigte Vorrichtung 10 dient der Bestimmung der optischen Wirkung einer optischen Linse 100, insbesondere eines Brillenglases. Die Vorrichtung 10 weist eine Anzeigeeinrichtung 20 auf, welche zum Anzeigen einer Teststruktur 21 eingerichtet ist. Beispielsweise kann es sich um einen Bildschirm oder ein Display handeln und verschiedene Teststrukturen anzeigen zu können.

Die Vorrichtung 10 weist ferner eine Bilderfassungseinrichtung 30 auf, die eingerichtet ist zum Erfassen von Bilddaten der Teststruktur 21 aus mehreren Blickpunkten 31, 31', 31" durch Abbildungsstrahlengänge 32, welche die Linse 100 durchsetzen. Die Abbildungsstrahlengänge aus den verschiedenen Blickpunkten können einerseits mit einer Kamera nacheinander aufgenommen werden, welche nacheinander an den verschiedenen Positionen angeordnet wird. Vorzugsweise sind jedoch mehrere Kameras vorgesehen, um die Bilddaten parallel zu erfassen. Es versteht sich, dass auch Mischformen vorgesehen sein können. Beispielsweise kann eine Bilderfassungseinrichtung 30 eine erste Kamera 33 und eine zweite Kamera 34 aufweisen, wobei die erste Kamera 33 zum Erfassen von ersten Bilddaten aus einem ersten Blickpunkt 33 eingerichtet ist und die zweite Kamera 34 zum Erfassen von zweiten Bilddaten aus einem zweiten Blickpunkt 33" eingerichtet ist. Das Messvolumen 200 liegt zwischen der auf der Anzeigeeinrichtung 20 anzeigebaren Teststruktur 21 und der Bilderfassungseinrichtung 30.

Die Vorrichtung 10 weist ferner eine Recheneinheit 40 auf. Bei der Recheneinheit 40 kann es sich beispielsweise um einen Computer, Mikrokontroller, FPGA oder dergleichen handeln. Die Recheneinheit 40 ist eingerichtet zum Bestimmen einer dreidimensionalen Form der Linse 100 basierend auf den Bilddaten und zum Berechnen einer optischen Wirkung der Linse 100 basierend auf der dreidimensionalen Form. Mit anderen Worten wird also ein zweistufiges Vorgehen vorgeschlagen, bei welchen zunächst die dreidimensionale Form der Linse bestimmt wird und erst nachfolgend die optische Wirkung der Linse aus deren dreidimensionaler Form berechnet wird.

Dieser Ansatz gemäß der vorliegenden Offenbarung soll nachfolgend bezugnehmend auf die Fig. 2 bis 6 näher erläutert werden.

Fig. 2 zeigt eine Draufsicht einer durch eine Linse 100 aufgenommenen Teststruktur 21 einer Anzeigeeinrichtung 20. Hierbei kann es sich beispielsweise um die mit der Kamera 33 durch die Linse 100 aufgenommene Teststruktur 21 gemäß Fig. 1 handeln. Die Teststruktur 21 wird durch die Linse 100 verzerrt wiedergegeben. Aus einer solchen Ablenkung der Strahlen können bereits Rückschlüsse über die optische Wirkung der Linse 100 gezogen werden. Allerdings kann nur eine Aussage über die Wirkung der Linse 100 in ihrer Gesamtheit getroffen werden.

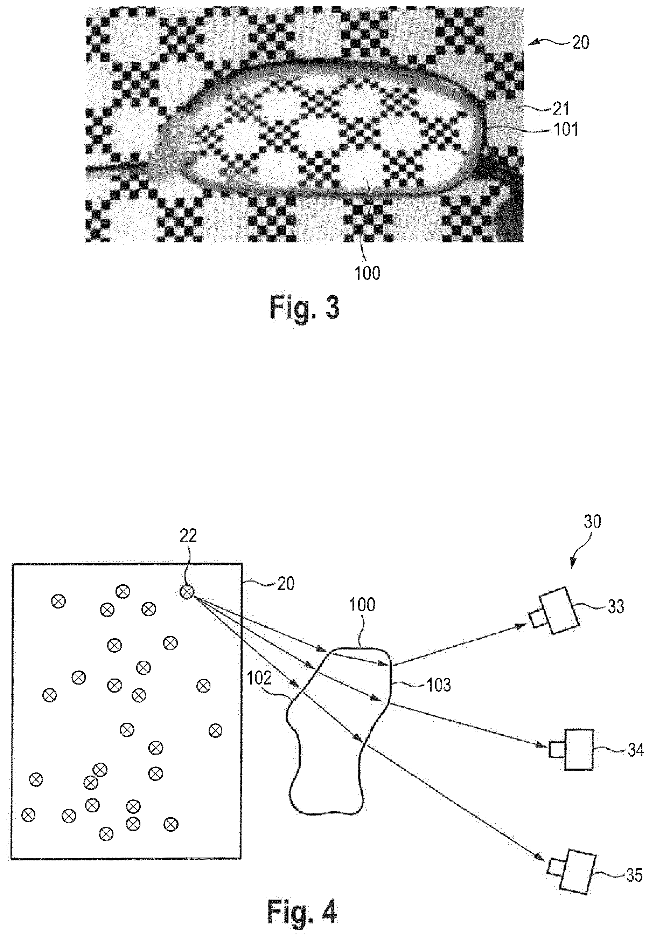

Fig. 3 zeigt ein weiteres beispielhaftes Bild, welches mit einer Kamera aufgenommen ist. Hierbei ist die Brille 101 mit der optischen Linse 100 jedoch stark verkippt im Messbereich angeordnet, so dass die durch die optische Linse 100 hervorgerufene Strahlablenkung die tatsächliche optische Wirkung in einer Trageposition nur begrenzt genau wiedergibt.

Fig. 4 zeigt eine beispielhafte Darstellung von Strahlengängen durch ein transparentes Objekt, wie beispielsweise eine Linse 100. Der Ursprungsort der Strahlengänge ist ein definierter Punkt 22 auf der Anzeigeeinrichtung 21. Die von dem definierten Punkt 22 ausgehenden Strahlengänge treten auf der Oberfläche 102 in die Linse 101 ein und an der Oberfläche 103 aus der Linse aus. Sie durchdringen die Linse 100 somit. Sowohl an der Eintrittsoberfläche 102, als auch an der Austrittsoberfläche 103, werden die Lichtstrahlen gebrochen. Je nach der räumlichen Lage bzw. Orientierung der Linse 100 relativ zur Anzeigeeinrichtung 20 und zur Bilderfassungseinrichtung 30, kann sich eine unterschiedliche optische Wirkung ergeben.

Die Erfinder haben erkannt, dass eine solche Unbestimmtheit bzw. Mehrdeutigkeit der optischen Wirkung aufgelöst werden kann, indem die Teststruktur aus mehreren Blickpunkten aufgenommen wird und somit eine Vielzahl an Abbildungsstrahlengängen erfasst wird (siehe auch Fig. 1), aus welchen sich wiederum die optischen Eigenschaften einer dazwischenliegenden Linse bestimmen lassen. Mit anderen Worten kann für die Abbildungsstrahlengänge ein Gleichungssystem mit einer Vielzahl von Gleichungen aufgestellt werden, welche jeweils eine Zuordnung zwischen aus unter mehreren Blickpunkten in die Bilderfassungseinrichtung eintretenden Abbildungsstrahlen, welche die Linse 100 durchsetzen, und deren bekannten Ursprungsorten auf der Anzeigeeinrichtung 20 hergestellt werden. Hieraus können wiederum die dreidimensionale Form der Linse 100 und auch optional ihr Brechungsindex bzw. eine Bremskraftverteilung innerhalb der Linse bestimmt werden.

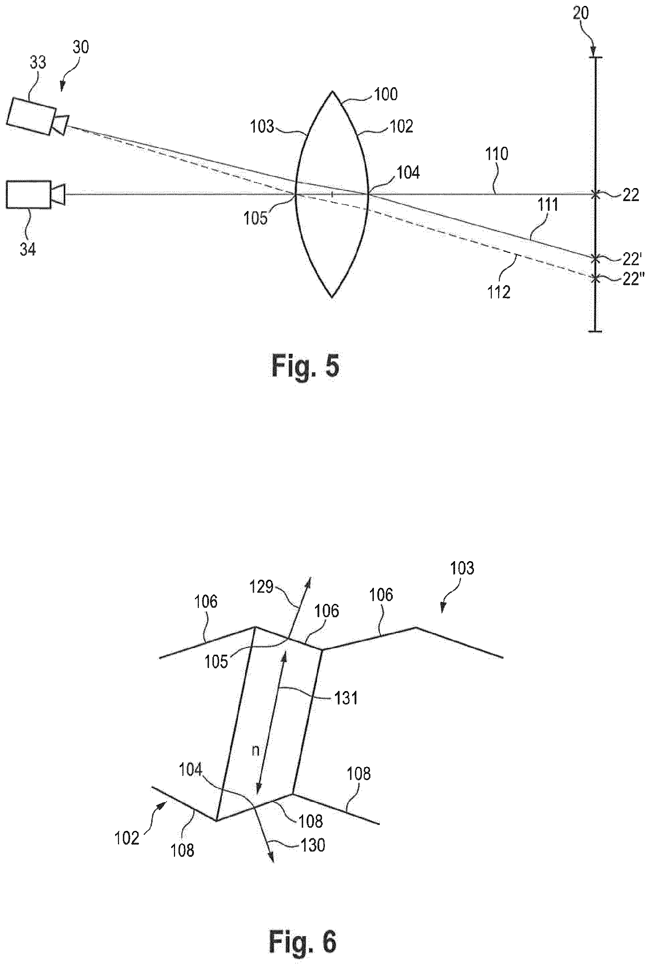

Ein vereinfachtes Beispiel von Strahlengängen durch eine Linse 100 ist in Fig. 5 wiedergeben. Der Bildpunkt 22 auf der Anzeigeeinrichtung 20 wird von der Kamera 34 erfasst. Der Strahlengang 110 tritt im Punkt 104 auf der Vorderfläche 102 Linse 100 in die Linse ein und im Punkt 106 auf der Rückfläche 103 aus der Linse aus. Bei Messung mit nur einer Kamera liegt somit vereinfacht gesprochen eine Gleichung vor, welche zwei Unbekannte aufweist, den Eintrittspunkt 104 und den Austrittspunkt 105 einschließlich der räumlichen Orientierung der Oberfläche an diesen Punkten. Indem die Bilderfassungseinrichtung 30 die Teststruktur aus weiteren Blickpunkten aufnimmt, wie durch die weitere Kamera 33 angedeutet, können weitere Strahlengänge 111 und 112 erfasst werden. Beim Strahlengang 111 fällt ein Austrittspunkt 104 mit dem Strahlengang 110 der Kamera 34 zusammen. Beim Strahlengang 112 fällt ein Eintrittspunkt 105 mit dem Strahlengang 110 der Kamera 34 zusammen. Somit lassen sich eine Mehrzahl von Gleichungen aufstellen, aus welchen sich die Eigenschaften der zwischen der Anzeigeeinrichtung 20 und der Bilderfassungseinrichtung 30 angeordneten Linse 100 bestimmen lassen.

Hierfür kann die Recheneinheit dazu eingerichtet sein, die Linse 100 vorzugsweise als zusammengesetzte Fläche aus parametrierbaren Flächenelementen zu modellieren, wie in Fig. 6 beispielhaft gezeigt. Aus der Ablenkung der Strahlen in den Punkten 104 und 105 kann eine Orientierung der Flächenelemente 106, 107 der Vorder- und Rückfläche 102, 103 bestimmt werden. Optional kann auch eine weitergehende Aufteilung innerhalb der Linse 100 vorgenommen werden. Beispielsweise können weitere Grenzflächen innerhalb der Linse 100 bestimmt werden. Optional kann die Recheneinheit ferner dazu ausgebildet sein, einen Brechungsindex der Linse 100 oder auch eine räumlichen Brechzahlverteilung der Linse zu bestimmen.

Optional kann die Vorrichtung als Vorrichtung zum Vermessen einer räumlichen Brechzahlverteilung einer in einem Messvolumen angeordneten optischen Linse ausgebildet sein. Hierfür kann vorzugsweise eine Schnittstelle eingerichtet zum Empfangen von Linsengeometriedaten, welche eine dreidimensionale Form der Linse beschreiben, vorgesehen sein. In diesem Fall muss die Form der Linse nicht berechnet werden und kann als Eingabeparameter für die Berechnung der räumlichen Brechzahlverteilung der Linse basierend auf den Bilddaten und den Linsengeometriedaten dienen.

Bezugnehmend auf Fig. 5 und Fig. 6 kann die Recheneinheit zum Bestimmen der dreidimensionalen Form der Linse basierend auf einer Rückverfolgung der in die Bilderfassungseinrichtung eintretenden Lichtstrahlen eingerichtet sein. Die Richtungen, unter welcher die Lichtstrahlen 110, 111, 112 in die Kameras 33, 34 der Bilderfassungseinrichtung eintreten, sind bekannt. Hierfür kann die Bilderfassungseinrichtung, wie nachfolgend noch beschrieben, kalibriert werden. Ausgehend von der jeweiligen Kamera 33, 34 können die eingehenden Strahlen somit zurückverfolgt werden. Die Linse 100 befindet sich im Strahlengang zwischen der Bilderfassungseinrichtung bzw. den jeweiligen Kameras (mit bekannter Position) und der Teststruktur (mit bekannter Position). Ausgehend von einem Modell der Linse 100, kann dieses Modell durch die Recheneinheit sukzessive derart parametriert werden, dass die (bekannte) Teststruktur derart durch das Modell der Linse 100 abgebildet wird, dass sich die von der Bilderfassungseinrichtung erfassten Bilddaten ergeben. Zur Parametrierung können insbesondere die Ausrichtungen der die Linsenoberfläche bildenden Flächenelemente, hier repräsentiert durch die Oberflächennormalen 129, 130, angepasst werden und ein Abstand 131 der Flächenelemente variiert werden, sowie optional ein Brechungsindex n bzw. eine Brechungsindexverteilung innerhalb der Linse variiert werden.

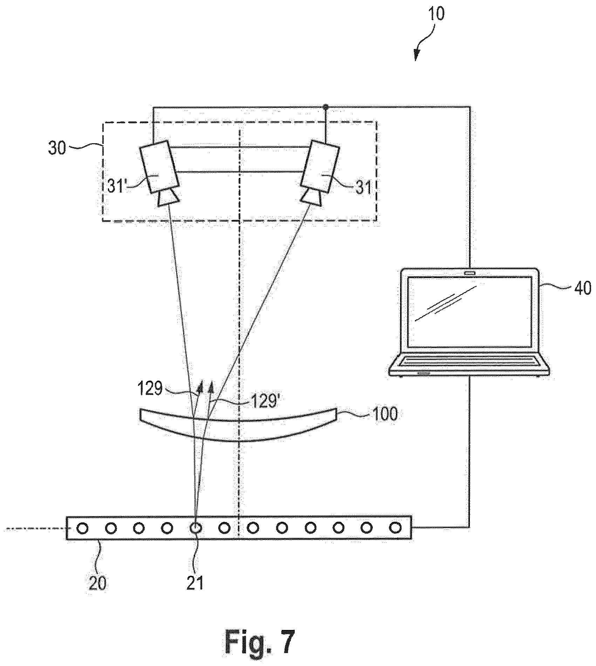

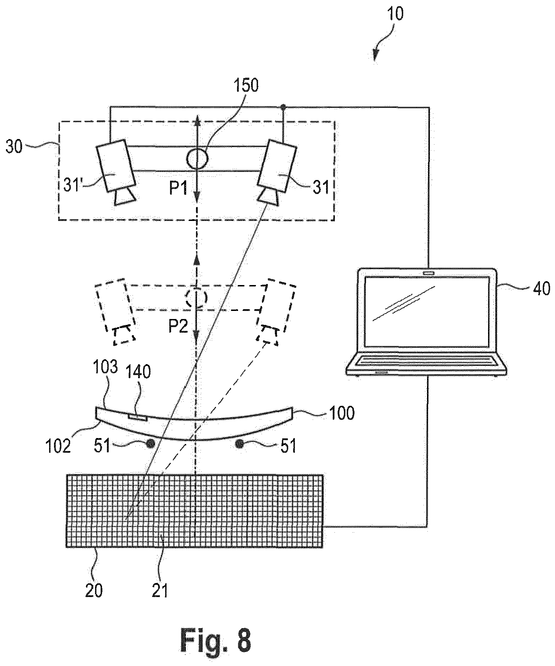

Fig. 7 und Fig. 8 zeigen weitere Ausführungsformen einer Vorrichtung 10 zum Vermessen der optischen Wirkung einer in einem Messvolumen angeordneten optischen Linse 100. Entsprechende Baugruppen werden mit den gleichen Bezugszeichen bezeichnet und zur Vermeidung von Wiederholungen nicht nochmals im Detail erläutert.

Fig. 8 zeigt eine Ausführungsform, bei welcher die Bilderfassungseinrichtung 30 zwei Kameras 30, 31' aufweist. Die Kameras sehen ein Muster bzw. eine Teststruktur 21 aus verschiedenen Blickwinkeln. Die Recheneinheit ist dazu ausgebildet, aus den entsprechenden Bilddaten das Messobjekt 4 zu rekonstruieren. Hierfür kann ein Gradientenfeld bestimmt werden aus den Oberflächenelementen bzw. aus den Normalen 129, 129', wie in Figs. 5 und 6 erläutert.

Licht aus definierten Quellen an definierten Ursprungspunkten der Teststruktur 21 durchdringt die Linse 100 und wird von der Bilderfassungseinrichtung 30 mit einem kalibrierten Kamerasystem aus verschiedenen Blickwinkeln aufgenommen. Aus den entstandenen Bildern werden die brechenden Flächen des Körpers rekonstruiert.

Das Prinzip funktioniert sowohl mit einer, zwei oder mehreren Kameras. In einer vorteilhaften Ausführungsform kommen zwei Kameras zum Einsatz, da hierbei ein gutes Kosten- / Nutzenverhältnis erzielt werden kann. Um die Genauigkeit weiter zu steigern, können noch weitere Kameras zum Einsatz kommen.

Die Bilderfassungseinrichtung 30 bzw. die Kameras 31, 31' ist derart kalibriert, dass eine Funktion bekannt ist, mit der für jede Sensorkoordinate ein eindeutiger Hauptlichtstrahl (Kamerastrahl) aus Ursprung und Richtung in 3D abgeleitet werden kann. Diese Kalibrierung kann nach dem Stand der Technik erfolgen. Alternativ kann anstatt der oben beschriebenen Kamerakalibrierung ein bekanntes Optikdesign der Kamera und/oder eines verwendeten Objektivs in das Model der Kamera mit einbezogen werden.

Die Anzeigeeinrichtung 20 kann beispielsweise selbstleuchtende Quellen, wie in einem Array angeordnete Leuchtdioden, ein TFT oder LED Display, ein 3D Display, Laser-Quellen, ein Polarisations-Display, oder auch eine kollimierte, wahlweise strukturierte Beleuchtungseinheit aufweisen. Die Anzeigevorrichtung kann auch angeleuchtet sein. Beispielsweise kann eine angeleuchtete Anzeigevorrichtung Test-Charts (z.B. Punktmuster oder Karomuster), ein insbesondere regelmäßiges 3D Pattern, ein unbekanntes Feature-reiches flaches Bild (wobei Positionen während der Optimierung geschätzt werden können) oder auch eine unbekannte Feature-reiche 3D-Szene (Positionen werden während der Optimierung geschätzt) aufweisen.

Die Recheneinheit 40 kann für die Bestimmung der dreidimensionalen Form weitergehende Informationen nutzen. Zur Rekonstruktion der dreidimensionalen Form können insbesondere auch auf den bekannten Blickpunkten bzw. Positionen der Kameras, aus welchen die Bilddaten erfasst werden, und auf einer bekannten Position der Teststruktur basieren. Bei den Bilddaten kann es sich im vorliegenden Beispiel um Orte der Abbildungen von in die Kameras eintretenden Lichtstrahlen auf den Kameradetektoren handeln. Aus den Bilddaten und den bekannten Blickpunkten können die in die Bilderfassungseinrichtung eintretenden Lichtstrahlen berechnet werden. Als Grundlage kann eine Kalibrierung der Bilderfassungseinrichtung dienen.

Optional kann die Recheneinheit 40 ferner dazu eingerichtet sein, die dreidimensionale Form der Linse unter Berücksichtigung einer oder mehrerer Randbedingungen zu bestimmen. Beispielsweise kann ein Anlagepunkt oder Anschlag 51 vorgegeben sein. An diesem Punkt ist die Lage der Linse 100 bekannt und kann bei der Bestimmung der dreidimensionalen Form der Linse berücksichtigt werden. Ferner können Informationen wie eine Form einer Vorder- und/oder Rückfläche der Linse, ein Brechungsindex oder Material, etc. vorgegeben sein. Optional kann die Vorrichtung dazu ausgebildet sein, auf der Linse vorhandene Informationen, beispielsweise in Form einer Gravur oder eines Markers 140, auszulesen und bei der Bestimmung der dreidimensionalen Form und/oder bei der Berechnung der optischen Wirkung berücksichtigen.

Ein besonders vorteilhafter Anwendungsfall der vorliegenden Erfindung ist die Brillenglas-Vermessung, insbesondere die Vermessung von progressiven Brillengläsern - auch bekannt als Gleitsichtbrillengläser. Einfachere Brillengläser wie sphärische, asphärische, torische oder prismatische Gläser können jedoch ebenfalls mit der vorgeschlagenen Vorrichtung vermessen werden.

Optional kann die Recheneinheit eingerichtet sein zum Berechnen eines ISO Scheitelbrechwerts oder eines Scheitelbrechwerts in einer vorgegebenen Messgerätekonfiguration, um vergleichbare Daten bereitzustellen. Durch Bereitstellen trägerspezifischer Daten wie z.B. Abstand einer Pupille zum Brillenglas (Hornhaut-Scheitel-Abstand HSA) und dessen Lage (z.B. Fassungsscheibenwinkel oder Vorneigung) können Gebrauchs-Scheitelbrechwerte berechnet werden.

Optional können mehrere Messobjekte gleichzeitig im Messraum vermessen werden. Für den Fall, dass eine Brille mit einem linken und einem rechten Brillenglas vermessen werden, kann die Recheneinheit ferner dazu ausgebildet sein, eine Position und Lage der Brillengläser relativ zueinander zu bestimmen. Hieraus können weitere Informationen wie z.B. der Abstand der Sehkanäle berechnet werden. Als mehrere Messobjekte kann auch ein transparenter Körper mit Zonen verschiedener Wirkungen vorgesehen werden. Dies kann z.B. eine Brille mit zwei Linsen sein oder eine Linse mit mehreren Zonen sein - Bi-, Tri, oder Multifokallinse.

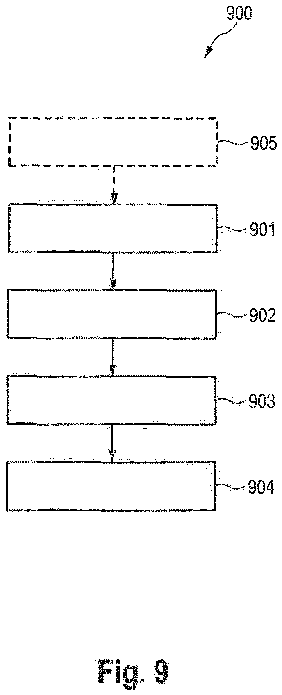

Fig. 9 zeigt ein Flussdiagramm eines Verfahrens 900 zum Vermessen der optischen Wirkung einer in einem Messvolumen angeordneten optischen Linse, insbesondere eines Brillenglases, mit den Schritten. In einem ersten Schritt 901 wird eine Teststruktur zur Anzeige auf einer Anzeigeeinrichtung bereitgestellt. In einem zweiten Schritt 902 werden Bilddaten der Teststruktur erfasst aus mehreren Blickpunkten durch Abbildungsstrahlengänge, welche die Linse durchsetzen. In einem dritten Schritt 903 wird eine dreidimensionale Form der Linse basierend auf Bilddaten (und den bekannten Positionen der Blickpunkte und der Anzeigeeinrichtung relativ zueinander) bestimmt. In einem vierten Schritt 904 wird eine optische Wirkung der Linse basierend auf deren dreidimensionaler Form berechnet. Die Berechnung kann für beliebige Gebrauchssituationen erfolgen. Mithin kann die Recheneinheit dazu eingerichtet sein, eine erste optische Wirkung entsprechend einem ISO Scheitelbrechwert zu berechnen und eine zweite optische Wirkung entsprechend einer Gebrauchssituation eines Nutzers zu berechnen.

Optional kann dem Messverfahren ein Schritt 905 zum Kalibrieren der Vorrichtung vorgelagert sein.

Ein entsprechendes Verfahren zum Kalibrieren der Vorrichtung kann seinerseits wiederum folgende Schritte aufweisen: In einem ersten Kalibierschritt wird eine Teststruktur auf der Anzeigeeinrichtung bereitgestellt. In einem zweiten Kalibrierschritt wird ein erster Abstand zwischen der Bilderfassungseinrichtung und der Anzeigeeinrichtung eingestellt und werden aus dem ersten Abstand Bilddaten der Teststruktur mit der Bilderfassungseinrichtung erfasst.

Wie in Fig. 8 gezeigt kann eine Höhenverstelleinrichtung, 150 vorgesehen sein, die dazu eingerichtet ist, einem Abstand zwischen der Bilderfassungseinrichtung und der Anzeigeeinrichtung zu variieren. In diesem Kontext kann die Recheneinheit ferner dazu eingerichtet sein, basierend auf aus unterschiedlichen Abständen zwischen der Bilderfassungseinrichtung und der Anzeigeeinrichtung erfassten Bilddaten eine Strahlrichtung, der von der Bilderfassungseinrichtung erfassten Lichtstrahlen, zu bestimmen.

In einem weiteren Schritt des Verfahrens zum Kalibrieren der Vorrichtung kann ein zweiter Abstand zwischen der Bilderfassungseinrichtung und der Anzeigeeinrichtung eingestellt werden und Bilddaten der Teststruktur mit der Bilderfassungseinrichtung aus dem zweiten Abstand erfasst. Hieraus kann in einem weiteren Schritt eine Richtung von einfallenden Lichtstrahlen, welche von der Bilderfassungseinrichtung erfasst werden, und korrespondierenden Bildpunkten in den Bilddaten bestimmt werden.

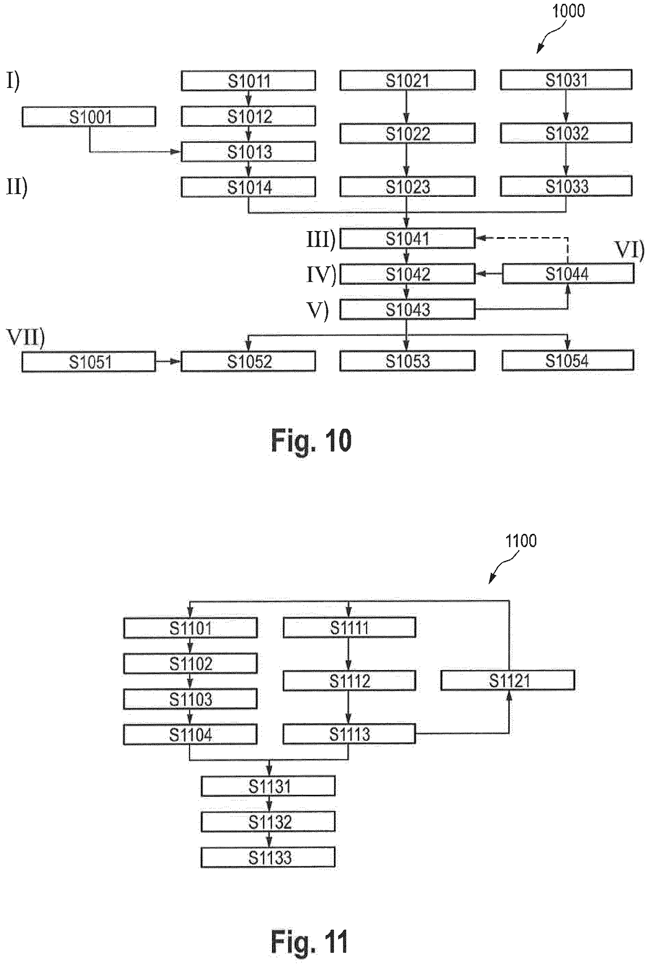

Fig. 10 zeigt ein detailliertes Flussdiagramm einer Ausführungsforme eines Verfahrens 1000 zum Vermessen der optischen Wirkung einer in einem Messvolumen angeordneten Linse.

In einem ersten Schritt S1011 wird eine Teststruktur auf der Anzeigeeinrichtung angezeigt. Beispielsweise kann es sich hierbei um ein gesamtes Punkte- oder Streifenmuster handeln. In einem weiteren Schritt S1012 werden Bilddaten der Teststruktur von der Bilderfassungseinrichtung erfasst. In Schritt S1013 können Positionen von Merkmalen der Teststruktur, beispielsweise die Positionen von Musterpunkten in den Bilddaten (entsprechend Positionen auf einer Detektorfläche der Bilderfassungseinrichtung), bestimmt werden. Hierbei kann in Schritt S1001 eine Kamerakalibrierung erfolgen, wie beispielsweise vorstehend erläutert oder in Fig, 11 im Detail beschrieben. In Schritt S1014 können dann in die Bilderfassungseinrichtung einfallende Lichtstrahlen bzw. deren Richtungen bestimmt werden. Aus den Kamerabildern des angezeigten Musters betrachtet durch die zu vermessende Linse können die Lichtstrahlen, welche in die Kamera einfallen, als 3D Vektor bestimmt werden.

In einem Schritt S1021 kann ein Voll- oder Teilmuster einer Teststruktur auf der Anzeigeeinrichtung angezeigt werden. In einem weiten Schritt S1022 werden Bilddaten der Teststruktur von der Bilderfassungseinrichtung erfasst. In Schritt S1023 kann eine Zuordnung von Musterpunkten zu Bildpunkten in den Bilddaten vorgenommen werden. Insbesondere kann eine Sequenz von unterschiedlichen Testmustern bereitgestellt werden, um eine etwaige Mehrdeutigkeit bei der Zuordnung von Musterpunkten zu Bildpunkten in den Bilddaten aufzulösen. Mit anderen Worten kann eine Zuordnung von Leuchtflecken in den mit der Bilderfassungseinrichtung erfassten Bilddaten zu einer Position der Leuchtpunkte auf Anzeigeeinrichtung und damit auch zu den berechneten Lichtstrahlen, welche in die Bilderfassungseinrichtung eingefallen sind erfolgen. Die Recheneinheit kann alternativ oder zusätzlich dazu eingerichtet sein, Nachbarschaftsbeziehungen aus einem Gesamtmuster einer Teststruktur zu bestimmen.

In einem Schritt S1031 kann auf der Anzeigeeinrichtung eine flächige Ausleuchtung bereitgestellt werden. Beispielsweise können alle Pixel der Anzeigeeinrichtung "weiß" anzeigen. Infolgedessen kann sich eine Kontur der Linse abzeichnen und in Schritt S1032 eine Kontur der Linse bestimmt werden. In einem Schritt S1033 können eine Lage und Abmaße der Linse basierend auf der erfassten Kontur bestimmt werden. Mit anderen Worten kann auf einfache Art und Weise eine Lage der Linse im Messvolumen bestimmt werden.

In Schritt S1041 kann eine Berechnung einer "bestpassenden" parametrisierbaren Linse erfolgen. Vorzugsweise kann durch Rückwärtspropagation der Kameralichtstrahlen eine "bestpassende" parametrisierbare Linse ermittelt werden, welche im Messvolumen des Geräts liegen könnte. Als parametrisierbare Linse wird eine Linse verstanden, welche sich mit wenigen Parametern wie Radius, Dicke, Brechungsindex beschreiben lässt. Hierzu gehören z.B. sphärische und torische Linsen. Torische Linsen sind ein allgemeiner Kompromiss, welcher hier angewendet werden kann. In einer spezielleren Ausführung kann es ausreichend sein, einzelne "torische Zonen" auf der Linse zu definieren und nur hier das Brillenglas zu beschreiben. Eine erste dieser Zonen kann z.B. ein "Fernbereich" eines Gleitsichtglases sein. Eine zweite dieser Zonen kann z.B. ein "Nahbereich" eines Gleitsichtglases sein. Weitere Parameter können neben dem Ort der Linse bzw. den einzelnen Flächen, die Radien, die Dicke und der Brechungsindex sein.

In Schritt S1042 kann eine Bestimmung einer "bestpassenden" Gradientenfläche der Vorder- und oder Rückfläche der Linse erfolgen durch inverses Raytracing der Kamerastrahlen. Eine Fläche der in Schritt S1041 bestimmten "bestpassenden" parametrisierbaren Linse kann somit als Gradientenfläche beschrieben werden und die Gradienten an den Orten der Strahldurchtritte so variiert werden, dass durch Rückwärtspropagation der Kamerastrahlen die Positionen der Leuchtpunkte auf dem Anzeigeeinrichtung perfekt getroffen werden. Vereinfacht gesprochen wird die dreidimensionale Form der Linse also derart angepasst, dass die von der Bilderfassungseinrichtung empfangenen Lichtstrahlen und die zugehörigen Strahlquellen auf der Anzeigeeinrichtung zusammenpassen.

In Schritt S1043 kann eine Vorder- und oder Rückfläche der Linse durch Integration aus der Gradientenfläche erhalten werden. Mit anderen Worten wird aus einer abschnittsweise bzw. für Flächenelemente bestimmten Gradientenfläche eine (durchgängige) neue Oberfläche bestimmt. Hierbei kann es sich um die Vorderfläche oder die Rückfläche der Linse handeln.

Gemäß Schritt S1044 können die Schritte S1042 und S1043 iterativ wiederholt werden. Beispielsweise können die Schritte so lange wiederholt werden, bis ein Gütekriterium erfüllt ist. Optional kann, wenn keine hinreichende Güte erreicht wird, auch der Schritt S1041 in die Iterationsschleife mit einbezogen werden, um alternative Linsengeometrien zu berücksichtigen. Als Ergebnis der Iteration kann eine dreidimensionale Form der Linse vorliegen.

In einer weiteren Ausführungsform kann eine Form der Linse vorgegeben sein und stattdessen eine räumliche Brechzahlverteilung innerhalb der Linse analog iterativ bestimmt werden.

Aus der bestimmten dreidimensionalen Form (ggf. inkl. des Brechungsindices) können nachfolgend eine oder mehrere Größen bestimmt werden. In Schritt S1052 kann ein Gebrauchswert, insbesondere ein nutzerspezifischer Gebrauchswert, berechnet werden. Hierfür können in Schritt S1051 trägerspezifische Daten bereitgestellt werden, wie z.B. ein Abstand Hornhaut zu Scheitel. In Schritt S1053 kann ein ISO Scheitelbrechwert bestimmt werden. In Schritt S1054 kann ein Scheitelbrechwert in einer Gerätekonfiguration bestimmt werden.

Wurden mehrere Linsen oder Brillengläser gleichzeitig im Messvolumen angeordnet, so können optional zusätzliche Parameter wie z.B. der Abstand der Progressionskanäle bestimmt werden.

Es versteht sich, dass die vorstehend genannten Schritte durch die Recheneinheit durchgeführt werden können und diese entsprechend zur Durchführung der Schritte eingerichtet sein kann.

Fig. 11 zeigt ein Flussdiagramm einer beispielhaften Ausführungsform eines Verfahrens 1100 zum Kalibrieren einer Vorrichtung zum Vermessen von individuellen Daten einer in einem Messvolumen angeordneten optischen Linse. Die Kalibrierung kann insbesondere dazu dienen, einen Funktionensatz bereitzustellen, welcher einem - vorzugsweise jedem - Bildpunkt der von der Bilderfassungseinrichtung erfassten Bilddaten eine Strahlrichtung wie beispielsweise einen 3D Vektor zuordnet, welcher eine Strahlrichtung bzw. einen Lichtstrahl beschreibt, der in die Bilderfassungseinrichtung eindringt. Ein solcher Funktionensatz kann wie folgt aussehen:

$$\vec{r}(x,y) = \begin{pmatrix} x_0 \\ y_0 \\ 0 \end{pmatrix} + \alpha \begin{pmatrix} dx \\ dy \\ 1 \end{pmatrix}$$

wobei $(x_0, y_0, 0)$ einen Punkt des Lichtstrahls einer Bezugsebene der Bilderfassungseinrichtung, vorzugsweise einen Punkt des Lichtstrahls einer Bezugsebene im Linsensystem einer Kamera der Bilderfassungseinrichtung beschreibt und $(dx, dy, 1)$ den Richtungsvektor des einfallenden Strahles. Folglich besteht der Funktionensatz aus den vier Funktionen $x_0(x,y)$, $y_0(x,y)$, $dx(x,y)$ und $dy(x,y)$ wobei $x$ und $y$ die Pixelkoordinaten in Bilddaten der Bilderfassungseinrichtung, hier einer Kamera, beschreiben.

Ein derartiger Funktionensatz kann bestimmt werden, indem eine Teststruktur, beispielsweise ein Punktemuster, auf der Anzeigeeinrichtung angezeigt wird und dieses mit den Kameras der Bilderfassungseinrichtung unter verschiedenen Abständen betrachtet wird. Zu diesem Zweck kann die Vorrichtung, wie in Fig. 8 beispielhaft dargestellt, eine Höhenverstelleinrichtung 150 aufweisen, die dazu eingerichtet ist, einen Abstand zwischen der Bilderfassungseinrichtung und der Anzeigeeinrichtung zu variieren.

Bei dem in Fig. 11 gezeigten Verfahren können die Schritte S1101 bis S1104 jeweils den oben beschriebenen Schritten S1011 bis S1014 in Fig. 10 entsprechen. Die in Fig. 11 gezeigten Schritte S1111 bis S1113 können jeweils den oben beschriebenen Schritten S1021 bis S1023 entsprechen. Zusätzlich ist jedoch eine Schleife vorgesehen, wobei in Schritt S1121 ein Abstand zwischen der Bilderfassungseinrichtung und der Anzeigeeinrichtung variiert wird. Wie in Fig. 8 gezeigt ändert sich durch eine Variation des Abstandes eine Richtung der einfallenden Lichtstrahlen. Die Recheneinheit kann dazu eingerichtet sein, eine Richtung der einfallenden Lichtstrahlen basierend auf den im ersten Abstand erfassten Bilddaten und den im zweiten Abstand erfassten Bilddaten, zu bestimmen. Es versteht sich, dass die Bestimmung ferner auf einer Relativposition zwischen der Anzeigeeinrichtung und der Bilderfassungseinrichtung sowie der die Variation des Abstands basieren kann.

Wie in Fig. 11 gezeigt, kann in einem Schritt S1131 eine Interpolation von 3D Lichtstrahlen von einer Mehrzahl von, vorzugsweise für jeden Bildpunkt oder Pixel der Bilderfassungseinrichtung vorgenommen werden. Nachfolgend kann in Schritt S1132 eine Bestimmung der Größen $x_0$, $y_0$, $dx$ und $dy$ für die Bildpunkte vorgenommen werden. In einem nächsten Schritt S1133 kann ein Polynomfit an die Größen $x_0$, $y_0$, $dx$ und $dy$ durchgeführt werden. Im Ergebnis kann damit ein Funktionssatz bestimmt werden, welcher jedem Bildpunkt der von der Bilderfassungseinrichtung erfassten Bilddaten eine Richtung eines einfallenden Lichtstrahls zuordnet.

Optional kann analog zur Kamerakalibrierung eine räumliche Lage eines Auflagepunktes 51, wie beispielhaft in Fig. 8 dargestellt, bestimmt werden. Hierfür kann die Anzeigeeinrichtung 20 dazu eingerichtet sein, als Teststruktur einen einfarbigen bzw. Hintergrund mit konstanter Helligkeit anzuzeigen. Die Bilderfassungseinrichtung erfasst die Anzeige ohne eingelegte Linse. Die Auflagepunkte führen in diesem Fall zu einem Schattenwurf (z.B. Kreis bei Auflagekugeln), welcher durch die Bilderfassungseinrichtung erfasst und in den Bilddaten enthalten ist. Die Recheneinheit kann dazu ausbildet sein, basierend auf diesen Bilddaten eine Position der Auflagepunkte 51 zu bestimmen. Diese können wiederum als Randbedingungen bei der Bestimmung einer dreidimensionalen Form einer Linse berücksichtigt werden. Bei der Betrachtung eines Auflagepunktes von mindestens zwei Kameras kann dessen zentraler Punkt (die Lage) durch einen Schnitt der Kamerastrahlen ermittelt werden. Die Position der Auflagenpunkte kann verwendet werden, um einem Algorithmus, welcher die Form des Brillenglases bestimmt, einen Erwartungswert für dessen Position im Messvolumen zuzuordnen. Ein Vorteil dieser Ausgestaltung besteht in einer verbesserten Genauigkeit.

Es versteht sich, dass die obigen Erläuterungen für die nachfolgenden Ausführungsbeispiele entsprechend gelten können und umgekehrt. Um Wiederholungen zu vermeiden soll im Folgenden soll insbesondere auf weitergehende Aspekte eingegangen werden. Merkmale der obigen und nachfolgenden Ausführungsbeispiele können vorteilhaft miteinander kombiniert werden.

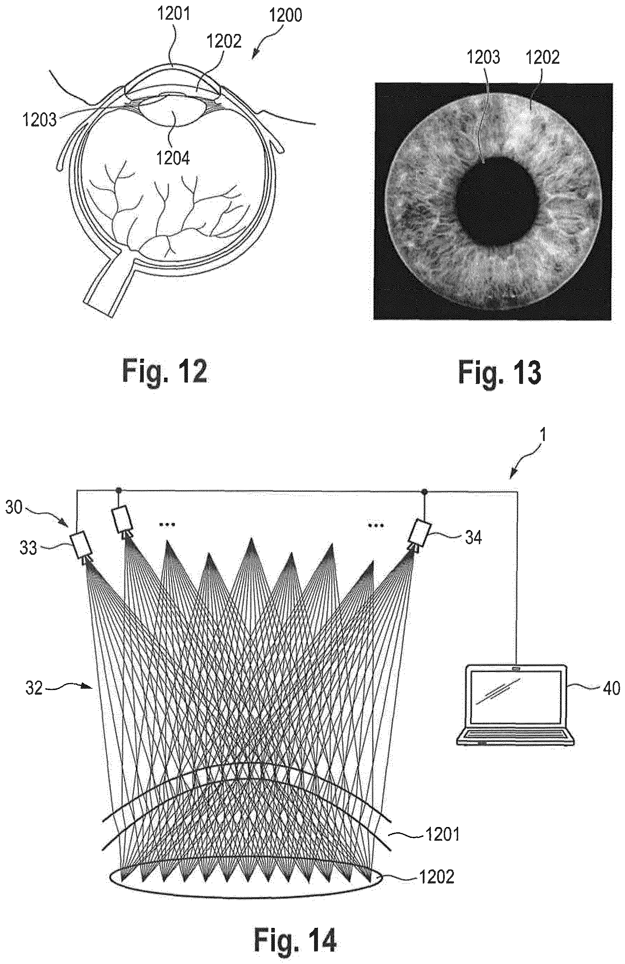

Die Erfinder haben erkannt, dass die hierin beschriebenen Konzepte auch vorteilhaft zur Cornea-Vermessung eingesetzt werden können. Fig. 12 zeigt eine schematische Schnitt-Darstellung eines Auges 1200. Das Auge weist eine Cornea oder Hornhaut 1201, eine Iris 1202, eine Pupille 1203 und die Linse 1204 auf. Fig. 13 zeigt eine Draufsicht auf das Auge mit einer Abbildung der Iris 1202 und der Pupille 1203. Ein wesentlicher Beitrag zur Fehlsichtigkeit kommt hierbei jedoch nicht von der Linse 1204 sondern von der Cornea 1201. Ein wesentlicher Beitrag zur Fehlsichtigkeit eines Probanden kann einer Hornhautverkrümmung geschuldet sein. Es ist daher wünschenswert, eine Form der Hornhaut objektiv bestimmen zu können.

Fig. 14 zeigt eine schematische Darstellung einer Vorrichtung zur Cornea-Vermessung eines Probanden gemäß einem weiteren Aspekt der vorliegenden Offenbarung. Die Vorrichtung kann folgendes aufweisen: eine Bilderfassungseinrichtung (30) eingerichtet zum Erfassen von Bilddaten einer Iris (1202) des Probanden aus mehreren (bekannten) Blickpunkten durch Abbildungsstrahlengänge (32), welche die Cornea (1201)durchsetzen; und eine Rechnereinheit (40). Die Recheneinheit ist eingerichtet ist zum: Bereitstellen eines mathematischen Modells eines vorderen Augenabschnitts des Probanden mit einem mathematischen Modell der Cornea und der Iris; Identifizieren und Registrieren von Bildmerkmalen der Iris welche in mehreren Bildern der Bilddaten vorhanden sind; Bestimmen von Abweichungen zwischen tatsächlichen Positionen der Bildmerkmale der Iris in den aus mehreren Blickpunkten erfassten Bildern und erwarteten Positionen der Bildmerkmale der Iris in den aus mehreren Blickpunkten erfassten Bildern unter Berücksichtigung des mathematische Modells der Cornea und der Lage der Iris; Adaptieren von Parametern des mathematischen Modells der Cornea derart, dass die Abweichungen minimiert werden; und Bestimmen einer Messgröße der Cornea aus dem adaptierten mathematischen Modell der Cornea.

Die Bilderfassungseinrichtung 30 kann erneut gleich oder ähnlich ausgestaltet sein, wie für Fig. 1 beschreiben. Die Bilderfassungseinrichtung erfasst Bilddaten der Iris 1202, wobei die Strahlengänge durch die Cornea 1201 hindurchtreten. Zur Erfassung der Bilddaten aus verschiedenen bekannten Blickpunkten kann eine Kamera 33 nacheinander an verschiedenen bekannten Positionen positioniert werden. Hierfür kann eine Positioniereinrichtung (nicht gezeigt) vorgehen sein. Alternativ oder zusätzlich können mehrere Kameras 33, 34 vorgesehen sein, welche die Bilddaten parallel erfassen. Ein Vorteil der parallelen Erfassung besteht darin, dass sich das Auge des Nutzers nicht zwischen den verschiedenen Messungen bewegt.

Die Erfinder haben erkannt, dass die zwischen der Iris 1202 und der Bilderfassungseinrichtung 30 liegende Cornea 1201 auch ohne Kenntnis des Aussehens der Iris 1202 berechnen lassen kann. Zwar weist die Iris 1202 eine unbekannte Struktur bzw. ein unbekanntes Muster auf. Allerdings ist die Iris 1202 üblicherweise stark strukturiert. Die Erfinder haben erkannt, dass eine Vielzahl von Bildmerkmalen der Iris identifiziert und nachfolgend hinsichtlich ihrer Position in mehreren aus unterschiedlichen Positionen aufgenommenen Bildern der Bilddaten ausgewertet werden können. Hierfür können aus den Abbildungsstrahlengängen 32, welche an den jeweiligen bekannten Positionen erfasst werden, ein Gleichungssystem aufgestellt werden, aus welche sich die Form der Cornea 1201 berechnen lässt.

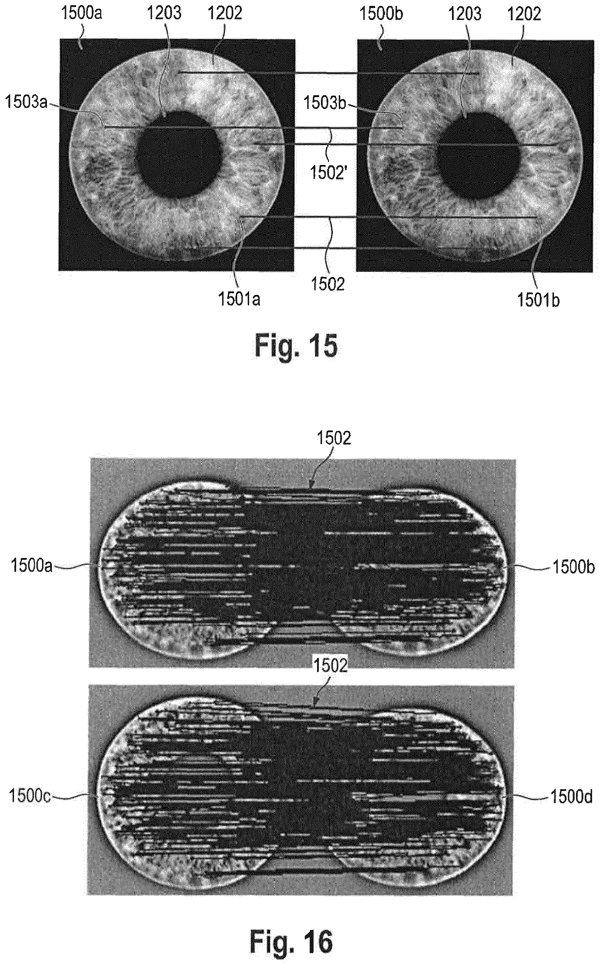

Fig. 15 und Fig. 16 zeigen die Zuordnung bzw. Korrelation von unbekannten Bildmerkmalen in aus unterschiedlichen Blickpunkten aufgenommenen Bildern der Iris. Das linke Bild in Fig. 15 zeigt eine erste Abbildung 1500a der Iris 1202 - durch die Cornea hindurch - aus einer ersten Position, beispielsweise aufgenommen mit der Kamera 33 in Fig. 14. Das rechte Bild in Fig. 16 zeigt eine zweite Abbildung 1500b der iris 1202 - durch die Cornea hindurch - aus einer zweiten Position, beispielsweise aufgenommen mit der Kamera 34 in Fig. 14. Da es sich bei der Iris um eine üblicherweise strakt strukturierte Fläche handelt, kann eine Korrelation 1502 zwischen gleichen Ausgangspunkten 1501a und 1501b der Iris 1201 bestimmt werden. Ein weiteres Beispiel für korrespondierende Punkte ist durch Bezugszeichen 1503a und 1503b verbunden durch 1502' angegeben. Eine derartige Zuordnung 1500 kann für eine Vielzahl an Bildern 1500a bis 1500d und Bildpunkten vorgenommen werden, wie in Fig. 16 gezeigt.

Basierend auf dieser Korrelations- bzw. Zuordnungsanalyse können eine Vielzahl an Strahlengängen rekonstruiert werden, wie in Fig. 14 gezeigt. Wie aus den gezeigten Strahlengängen 32 ersichtlich, treten die Lichtstrahlen ausgehend von einem gleichen Bildpunkt auf der Iris 1202 an unterschiedlichen Punkten durch die Cornea 1201 hindurch und werden an verschiedenen Orten von der Bilderfassungseinrichtung 30 erfasst.. Wenn nun ein gleicher Ausgangspunkt in den Abbildungen identifiziert ist, können Aussagen über die, zwischen dem Ausgangspunkt auf der Iris 1202 und dem Eintritt in die Kameras 33, 34 liegende, Cornea 1201 getroffen werden, wie vorstehend mit Bezug auf Fig. 14 beschrieben.

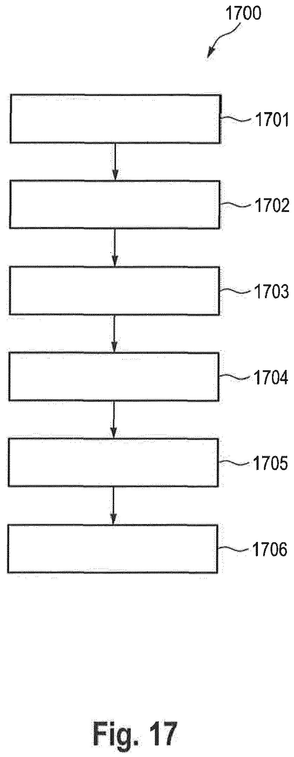

Fig. 17 zeigt ein Flussdiagram einer Ausführungsform eines Verfahrens zur Cornea-Vermessung eines Probanden. In einem optionalen vorgelagerten Schritt kann das Kamerasystem kalibriert werden. Die Kalibrierung kann jedoch bereits vom Hersteller vorgenommen sein. In einem ersten Schritt 1701 können Bilddaten einer Iris des Probanden aus mehreren Blickpunkten erfasst werden durch Abbildungsstrahlengänge, welche die Cornea durchsetzen. In einem zweiten Schritt 1702 kann ein mathematisches Modell eines vorderen Augenabschnitts des Probanden mit einem mathematischen Modell der Cornea (und der Lage der Iris relativ zur Cornea) bereitgestellt werden. In einem dritten Schritt 1703 können Bildmerkmale der Iris welche in mehreren (vorzugsweise allen) Bildern der Bilddaten vorhanden sind identifiziert und registriert (bzw. in den Bildern zugeordnet werden). In einem vierten Schritt 1704 können Abweichungen zwischen tatsächlichen Positionen der Bildmerkmale der Iris in den aus mehreren Blickpunkten erfassten Bildern und erwarteten Positionen der Bildmerkmale der Iris in den aus mehreren Blickpunkten erfassten Bildern unter Berücksichtigung des mathematische Modells der Cornea und der Lage der Iris bestimmt werden. In einem fünften Schritt 1705 können Parametern des mathematischen Modells der Cornea derart angepasst werden, dass die Abweichungen minimiert werden. Die Schritte 1703 und 1704 können vorzugsweise iterativ wiederholt werden. In einem sechsten Schritt kann nun eine Messgröße der Cornea aus dem adaptierten mathematischen Modell der Cornea bestimmt werden. Beispielsweise kann eine Brechkraft oder ein Astigmatismus ausgewertet werden.

Zusammenfassend können die hierin offenbarten Lösungen auf dem Gebiet der Augenoptik insbesondere eine vereinfachte berührungslose Vermessung von in einem Messvolumen angeordneten Linsenelementen oder auch eine berührungslose Vermessung der Cornea ermöglichen, insbesondere bei verminderter Beeinträchtigung eines lichtempfindlichen Nutzers.

## Patentansprüche

1. Vorrichtung (10) zum Vermessen der optischen Wirkung einer in einem Messvolumen (200) angeordneten optischen Linse (100), insbesondere eines Brillenglases, mit

   - einer Anzeigeeinrichtung (20) eingerichtet zum Anzeigen einer Teststruktur (21);
   - einer Bilderfassungseinrichtung (30), eingerichtet zum Erfassen von Bilddaten der Teststruktur aus mehreren Blickpunkten (31, 31', 31") durch Abbildungsstrahlengänge (32), welche die Linse (100) durchsetzen; und
   - einer Recheneinheit (40), wobei die Recheneinheit eingerichtet ist zum:

   - Bestimmen einer dreidimensionalen Form der Linse (100) basierend auf den Bilddaten; und
   - Berechnen einer optischen Wirkung der Linse (100) basierend auf deren dreidimensionaler Form.

2. Vorrichtung nach Anspruch 1, **dadurch gekennzeichnet, dass** die Bilderfassungseinrichtung (30) eine erste Kamera (33) und eine zweite Kamera (34) aufweist, wobei die erste Kamera (33) zum Erfassen von ersten Bilddaten aus einem ersten Blickpunkt eingerichtet ist und die zweite Kamera (34) zum Erfassen von zweiten Bilddaten aus einem zweiten Blickpunkt eingerichtet ist; und wobei die Recheneinheit (40) eingerichtet ist zum Bestimmen der dreidimensionalen Form der Linse (100) basierend auf den ersten und zweiten Bilddaten.

3. Vorrichtung nach einem der vorhergehenden Ansprüche, **dadurch gekennzeichnet, dass** die Linse (100) ein Brillenglas ist und die Berechnung der optischen Wirkung des Brillenglases für eine Trageposition eines Nutzers erfolgt.

4. Vorrichtung nach einem der vorhergehenden Ansprüche, **dadurch gekennzeichnet, dass** die Recheneinheit (40) dazu eingerichtet ist, die dreidimensionale Form der Linse (100) iterativ mittels eines Integrationsverfahrens zu bestimmen.

5. Vorrichtung nach einem der vorhergehenden Ansprüche, **dadurch gekennzeichnet, dass** die Recheneinheit (40) eingerichtet ist zum Bestimmen der dreidimensionalen Form der Linse (100) basierend auf einer Rückverfolgung der in die Bilderfassungseinrichtung (30) eintretenden Lichtstrahlen.

6. Vorrichtung nach einem der vorhergehenden Ansprüche, **dadurch gekennzeichnet, dass** das Bestimmen der dreidimensionalen Form der Linse (100) eine Aufteilung einer Vorder- und/oder Rückfläche (102, 103) der Linse in Flächenelemente (106, 108) und eine Bestimmung einer Ausrichtung der Flächenelemente, insbesondere eine Bestimmung von Oberflächennormalen (129, 130) der Flächenelemente, umfasst.

7. Vorrichtung nach Anspruch 6, **dadurch gekennzeichnet, dass**, die Recheneinheit (40) dazu ausgebildet ist, basierend auf der Ausrichtung der Flächenelemente eine dreidimensionale Form einer Vorderfläche (102) und einer Rückfläche (103) der Linse (100) zu bestimmen.

8. Vorrichtung nach einem der vorhergehenden Ansprüche, **dadurch gekennzeichnet, dass**, die Recheneinheit (40) dazu eingerichtet ist, die dreidimensionale Form der Linse (100) zu bestimmen unter Berücksichtigung der Randbedingung, dass eine Vorderfläche (102) oder Rückfläche (103) der Linse eine parametrisierbare Fläche, insbesondere eine Ebene, Sphäre, Torus oder ein Abschnitt davon ist.

9. Vorrichtung nach einem der vorhergehenden Ansprüche, **dadurch gekennzeichnet, dass**, die Recheneinheit (40) dazu eingerichtet ist, die dreidimensionale Form der Linse (100) ferner unter Berücksichtigung eines oder mehrerer bekannten Auflagepunktes (51) der Linse zu bestimmen; und/oder die Recheneinheit (40) dazu eingerichtet ist, die dreidimensionale Form der Linse unter Berücksichtigung einer Randbedingung zu bestimmen, wobei die Randbedingung bestimmt wird durch Auslesen von Information über die zu vermessende Linse (100), insbesondere durch Auslesen einer Markierung oder eines Codes (140) auf der Linse.

10. Vorrichtung nach einem der vorhergehenden Ansprüche, **dadurch gekennzeichnet, dass**, die Recheneinheit (40) ferner eingerichtet ist zur Bestimmung eines Brechungsindex, insbesondere zur Bestimmung einer räumlichen Brechzahlverteilung, der zu vermessenden Linse (100).

11. Vorrichtung nach einem der vorhergehenden Ansprüche, **gekennzeichnet durch**

   - eine Höhenverstelleinrichtung (150), die dazu eingerichtet ist, einem Abstand zwischen der Bilderfassungseinrichtung (30) und der Anzeigeeinrichtung (20) zu variieren; und
   - wobei die Recheneinheit (40) ferner dazu eingerichtet ist, basierend auf aus unterschiedlichen Abständen zwischen der Bilderfassungseinrichtung und der Anzeigeeinrichtung erfassten Bilddaten eine Strahlrichtung der von der Bilderfassungseinrichtung erfassten Lichtstrahlen zu bestimmen.

12. Vorrichtung zum Vermessen einer räumlichen Brechzahlverteilung einer in einem Messvolumen (200) angeordneten optischen Linse, insbesondere eines Brillenglases, mit

   - einer Anzeigeeinrichtung (20) eingerichtet zum Anzeigen einer Teststruktur (21);
   - einer Bilderfassungseinrichtung (30) eingerichtet zum Erfassen von Bilddaten der Teststruktur aus mehreren Blickpunkten (31, 31', 31") durch Abbildungsstrahlengänge, welche die Linse (100) durchsetzen;
   - einer Schnittstelle eingerichtet zum Empfangen von Linsengeometriedaten, welche eine dreidimensionale Form der Linse beschreiben; und
   - einer Recheneinheit (40), wobei die Recheneinheit eingerichtet ist zum

      - Berechnen einer räumlichen Brechzahlverteilung der Linse (100) basierend auf den Bilddaten und den Linsengeometriedaten.

13. Verfahren (905) zum Kalibrieren einer Vorrichtung (10) zum Vermessen von individuellen Daten einer in einem Messvolumen (200) angeordneten optischen Linse (100), wobei das Verfahren folgende Schritte aufweist:

   Bereitstellen einer Teststruktur (21) auf der Anzeigeeinrichtung (20);
   - Einstellen eines ersten Abstandes (P1) zwischen der Bilderfassungseinrichtung und der Anzeigeeinrichtung und Erfassen von Bilddaten der Teststruktur mit der Bilderfassungseinrichtung aus dem ersten Abstand;
   - Einstellen eines zweiten Abstandes (P2) zwischen der Bilderfassungseinrichtung und der Anzeigeeinrichtung und Erfassen von Bilddaten der Teststruktur mit der Bilderfassungseinrichtung aus dem zweiten Abstand;
   - Bestimmen einer Richtung von einfallenden Lichtstrahlen (110, 111, 112), welche von der Bilderfassungseinrichtung (30) erfasst werden, und korrespondierenden Bildpunkten in den Bilddaten basierend auf den im ersten Abstand erfassten Bilddaten und den im zweiten Abstand erfassten Bilddaten.

14. Verfahren (900) zum Vermessen der optischen Wirkung einer in einem Messvolumen (200) angeordneten optischen Linse (100), insbesondere eines Brillenglases, mit den Schritten:

   - Bereitstellen einer Teststruktur (21) zur Anzeige auf einer Anzeigeeinrichtung (20);
   - Erfassen von Bilddaten der Teststruktur aus mehreren Blickpunkten (31, 31', 31") durch Abbildungsstrahlengänge (32), welche die Linse (100) durchsetzen
   - Bestimmen einer dreidimensionalen Form der Linse (100) basierend auf Bilddaten; und
   - Berechnen einer optischen Wirkung der Linse (100) basierend auf deren dreidimensionaler Form.

15. Computerprogrammprodukt, umfassend Befehle, die bei der Ausführung des Programms durch einen Computer diesen Veranlassen, das Verfahren (900) nach Anspruch 14 auszuführen.

Fig. 1

Fig. 2

Fig. 3

Fig. 4

Fig. 5

Fig. 6

**Fig. 7**

Fig. 8

900

905

901

902

903

904

# Fig. 9

1000

I)

| S1001 | | S1011 | | S1021 | | S1031 |

| | | S1012 | | | | |

| | | S1013 | | S1022 | | S1032 |

II)

| | | S1014 | | S1023 | | S1033 |

III) | S1041 | VI)

IV) | S1042 | ← | S1044 |

V) | S1043 |

VII)

| S1051 | → | S1052 | | S1053 | | S1054 |

## Fig. 10

1100

| S1101 | | S1111 |

| S1102 | | S1112 | | S1121 |

| S1103 |

| S1104 | | S1113 |

| S1131 |

| S1132 |

| S1133 |

## Fig. 11

**Fig. 12**

**Fig. 13**

**Fig. 14**

Fig. 15

Fig. 16

1700

1701

1702

1703

1704

1705

1706

Fig. 17

Europäisches Patentamt
European Patent Office
Office européen des brevets

**EUROPÄISCHER RECHERCHENBERICHT**

Nummer der Anmeldung

EP 18 16 8823

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 101 143 A1 (UNIV BRAUNSCHWEIG TECH CAROLO WILHELMINA [DE]) 16. September 2009 (2009-09-16) | 1-15 | INV. G01B11/245 G01M11/02 |
| Y | * Zusammenfassung * * Absätze [0001] - [0005], [0021] - [0028], [0031] - [0055] * * Ansprüche 1,5 * * Abbildungen 1-7 * ----- | 3,9 | |
| X,D | WO 2016/207412 A1 (ZEISS CARL AG [DE]; ZEISS CARL VISION INT GMBH [DE]) 29. Dezember 2016 (2016-12-29) | 1-3,5,9, 14,15 | |
| Y | * Zusammenfassung * * Seite 1, Zeile 5 - Seite 7, Zeile 21 * * Seite 9, Zeilen 16-30 * * Seite 13, Zeile 20 - Seite 30, Zeile 23 * * Abbildung 11 * ----- | 9 | |
| Y | US 2016/109362 A1 (KUBIAK ROLF [DE] ET AL) 21. April 2016 (2016-04-21) * Zusammenfassung; Abbildung 2 * * Absätze [0002], [0009], [0010] - [0018], [0027] - [0031] * ----- | 6,7 | RECHERCHIERTE SACHGEBIETE (IPC) G01M G01B G01N |
| Y | Markus C Knauer ET AL: "Measuring the refractive power with deflectometry in transmission", , 1. Januar 2008 (2008-01-01), XP055258550, Gefunden im Internet: URL:http://www.dgao-proceedings.de/downloa d/109/109_a24.pdf [gefunden am 2016-03-15] * das ganze Dokument * ----- | 6,7 | |

-/--

Der vorliegende Recherchenbericht wurde für alle Patentansprüche erstellt

| Recherchenort | Abschlußdatum der Recherche | Prüfer |
|---|---|---|
| München | 30. August 2018 | Poizat, Christophe |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

Seite 1 von 2

EP 3 561 446 A1

**EUROPÄISCHER RECHERCHENBERICHT**

Nummer der Anmeldung

EP 18 16 8823

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 2017/134275 A1 (EIDGENOSSISCHE TECHNISCHE HOCHSCHULE ZURICH [CH]) 10. August 2017 (2017-08-10) * das ganze Dokument * * Absätze [0002], [0018] - [0093], [0114] - [0147], [0157], [0180] - [0198], [0206], [0215], [0218], [0290] * * Abbildungen 1-6 * * Abbildung 12 * ----- | 3 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 30. August 2018 | Poizat, Christophe |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 18 16 8823

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-08-2018

| Im Recherchenbericht angeführtes Patentdokument | Datum der Veröffentlichung | Mitglied(er) der Patentfamilie | Datum der Veröffentlichung |
|---|---|---|---|
| EP 2101143 A1 | 16-09-2009 | KEINE | |
| WO 2016207412 A1 | 29-12-2016 | CA 2989262 A1 | 29-12-2016 |
| | | CN 107810400 A | 16-03-2018 |
| | | DE 102015211879 A1 | 29-12-2016 |
| | | EP 3314230 A1 | 02-05-2018 |
| | | JP 6368872 B1 | 01-08-2018 |
| | | KR 20180019123 A | 23-02-2018 |
| | | US 2018120198 A1 | 03-05-2018 |
| | | WO 2016207412 A1 | 29-12-2016 |
| US 2016109362 A1 | 21-04-2016 | US 2016109362 A1 | 21-04-2016 |
| | | US 2016110860 A1 | 21-04-2016 |
| WO 2017134275 A1 | 10-08-2017 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1238690 B1

- WO 2016207412 A1